# EUROPÄISCHE PATENTSCHRIFT

(11) **EP 1 194 434 B1**
(45) Veröffentlichungstag und Bekanntmachung des Hinweises auf die Patenterteilung: **05.03.2003**
(21) Anmeldenummer: 00936881.2
(22) Anmeldetag: 14.06.2000
(51) Int. Cl.: C07F 7/18, A61L 2/16

(54) **ANTIMIKROBIELLE SILOXANQUAT-FORMULIERUNGEN, DEREN HERSTELLUNG UND VERWENDUNG**
ANTIMICROBIAL SILOXANE QUAT FORMULATIONS AND THE PRODUCTION AND USE THEREOF
FORMULATIONS DE SILOXANES QUATERNAIRES ANTIMICROBIENNES, LEUR PRODUCTION ET LEUR UTILISATION

(30) Priorität: 19.06.1999 DE 19928127
(43) Veröffentlichungstag der Anmeldung: 10.04.2002
(73) Patentinhaber: Clariant GmbH, 65929 Frankfurt am Main (DE)
(72) Erfinder: RAAB, Klaus, D-84508 Burgkirchen (DE); BENDER, Walter, CH-3422 Kirchberg (CH)
(86) Internationale Anmeldenummer: EP0005438
(87) Internationale Veröffentlichungsnummer: WO00078770

(56) Entgegenhaltungen:
- WO-A-87/06587
- WO-A-99/03866

## Beschreibung

Die vorliegende Erfindung betrifft Formulierungen, bestehend aus quatemären Ammoniumverbindungen, die durch eine Trialkoxysilangruppe substituiert sind, im folgenden kurz Siloxanquats genannt, und speziellen Solventien sowie die Herstellung und Verwendung dieser Siloxanquat-Formulierungen.

In der Patentliteratur ist seit langem die Herstellung von quaternären Ammoniumverbindungen mit einem Trialkoxysifansubstituenten und einem oder zwei Fettalkylresten sowie die Verwendung dieser Verbindungen als Biozide bekannt. Der Trialkoxysilansubstituent verstärkt die Tendenz von quaternären Ammoniumverbindungen, permanent auf Oberflächen der unterschiedlichsten Art, wie beispielsweise Textilien aus Polyamid, Baumwolle oder Polyester, Holz, Papier, Glas, Kunststoffe oder Metalle aufzuziehen. Die mit diesen Verbindungen ausgerüsteten Oberflächen erhalten damit einen dauerhaften, wasserfesten, antimikrobiellen Schutz. So wird beispielsweise die Verbindung 3-(Trimethoxysilyl)-propyl-octadecyl-dimethylammoniumchlorid, gelöst in Methanol, zur antimikrobiellen Ausrüstung diverser Oberflächen kommerziell angeboten.

EP-A-0 108 853 beschreibt die Herstellung von 3-(Trimethoxysilyl)-propyl-didecylmethylammoniumchlorid aus 3-Chlorpropyltrimethoxysilan und Didecylmethylamin ohne Solvens oder in einem Solvens wie Methanol. Ohne nähere Ausführungen werden als geeignete Solventien für bakterizide oder fungizide Anwendungen Wasser, wassermischbare Lösemittel wie Alkohole, beispielsweise Methanol, Ethanol und Butanol, Methylcellosolve, Ethylcellosolve und Ketone wie Methylethylketon genannt. Für die Textilbehandlung werden als geeignete Solventien Kohlenwasserstoffe, chlorierte Kohlenwasserstoffe, Ether und Benzol alternativ aufgeführt.

WO-87/06587 offenbart die Herstellung von Verbindungen des Typs 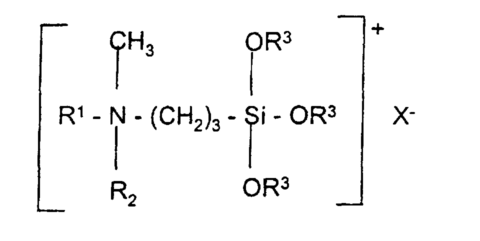 durch Umsetzung von tertiären Aminen mit 3-Chlorpropyltrialkoxysilanen unter Katalyse mit Alkaliiodid. Als Solvens bei dieser katalytischen Reaktion werden bestimmte Glykolether verwendet.

Weitere quaternäre Ammoniumverbindungen, unter anderem substituiert durch Trialkoxysilanreste, sind in US 4 883 917 aufgeführt. Sie werden bei Reaktionstemperaturen von 50 - 100°C entweder ohne Solvens oder in den in vielen Beispielen verwendeten Solventien Ethylacetat, Methylethylketon und para-Dioxan hergestellt. Als weitere mögliche Solventien für Quaternierungsreaktionen werden Tetrahydrofuran, Dimethoxyethan und Diglyme aufgeführt.

EP-A-0 415 540 beschreibt den Einsatz von quaternären Ammoniumverbindungen, substituiert durch Trialkoxysilanreste, für die antibakterielle und fungizide Ausrüstung von textilen Geweben zur Geruchseliminierung. Die bevorzugte Verbindung ist 3-(Trimethoxysilyl)-propyloctadecyl-dimethylammoniumchlorid. Ein Verfahren zur Herstellung dieser Verbindungen wird nicht offenbart, es wird nur auf den Stand der Technik bezug genommen.

In der Veröffentlichung K.J. Hüttinger, Chemiker-Zeitung, 111 (1987) Seite 213-220 wird die Verwendung von quaternären Trialkoxysilanen zur Herstellung von trägergebundenen Desinfektionsmitteln für die Wasserentkeimung beschrieben. Bei dieser Technik gelangt kein Wirkstoff in das Wasser im Gegensatz etwa zur Chlorierung oder Ozonisierung. Die Synthese der quaternären Trialkoxysilane erfolgt in Methanol oder Ethylenglykolmonomethylether bei 110°C im Autoklaven.

Die beim oben beschriebenen Stand der Technik während der Umsetzung von Halogenalkyltrialkoxysilanen mit tertiären Aminen eingesetzten Solventien oder nach der Umsetzung zugemischten Solventien besitzen den vor allem in der Textilindustrie großen Nachteil der leichten Entflammbarkeit und leichten Brennbarkeit. Viele der bisher eingesetzten Solventien wie beispielsweise Methanol haben einen unter den Reaktionstemperaturen liegenden Siedepunkt, so daß bei deren Einsatz während der Umsetzung unter erhöhtem Druck gearbeitet werden muß, was natürlich zu einem erhöhten apparativen und sicherheitstechnischen Aufwand führt. Quaternäre Ammoniumverbindungen mit Trialkoxysilansubstituenten sind, abhängig von der Alkylkettenlänge, meist Feststoffe. Da der Umgang mit und die Dosierung von Feststoffen aufwendiger ist als bei Flüssigkeiten, bevorzugen die meisten Anwender flüssige Formulierungen.

Es bestand daher die Aufgabe, flüssige Siloxanquat-Formulierungen zu entwickeln, die den Nachteil der leichten Entflammbarkeit und leichten Brennbarkeit nicht besitzen. Die eingesetzten Solventien müssen mit den Siloxanquats kompatibel sein, müssen die Endanwendung unterstützen und sollten günstige ökotoxikologische Eigenschaften besitzen. Ihre Einsetzbarkeit auch als Lösemittel während der Reaktion von Halogenalkyltrialkoxysilanen mit tertiären Aminen ist zwar wünschenswert, aber nicht unbedingt erforderlich. Außerdem bestand die Aufgabe, eine Siloxanquat-Formulierung zu entwickeln, die bereits in geringer Konzentration eine besonders hohe Wirksamkeit vor allem auf textilen Oberflächen gegen Mikroorganismen wie Bakterien und Pilze aufweisen, die der des Standes der Technik überlegen sein soll.

Diese Aufgaben werden gelöst durch das erfindungsgemäße Verfahren zur Herstellung der erfindungsgemäßen Siloxanquat-Formulierungen mit speziellen Glykolethern oder mit speziellen Dialkylglykolen als Solventien.

Gegenstand der Erfindung ist daher ein Verfahren zur Herstellung einer antimikrobiellen Formulierung, umfassend die Reaktion einer Verbindung der Formel 1

(R¹O)₃Si - (CH₂)₃ - X (1)

mit einer Verbindung der Formel 2

(H₃C)NR²R³ (2)

worin
- R¹: C₁-C₄-Alkyl
- R²: C₈-C₂₀-Alkyl
- R³: Methyl oder C₈-C₂₀-Alkyl
- X: Cl, Br bedeuten,
unter Ausschluß von lodiden im Molverhältnis von (1): (2) = 1:0,9 bis 1:1,4, gekennzeichnet durch die Durchführung der Reaktion in einem Lösungsmittel, welches der Formel 3 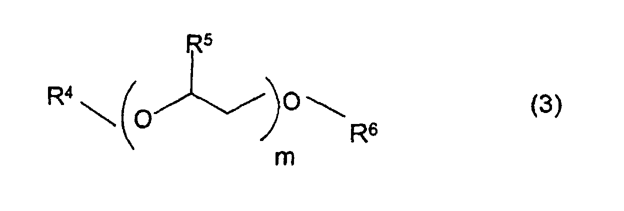 entspricht, worin
- R⁴: C₁-C₄-Alkyl
- R⁵: H oder Methyl
- R⁶: H oder Methyl
- m: 2, 3, 4 oder 5,
wobei für m = 2 aber R⁴ und R⁶ nicht gleichzeitig Methyl bedeuten,
und dieses Lösungsmittel nach der Reaktion nicht abgetrennt wird, oder die Zugabe dieses Lösungsmittels nach der Reaktion.

Ein weiterer Gegenstand der Erfindung ist eine antimikrobielle Formulierung, herstellbar nach dem oben beschriebenen Verfahren.

In einer bevorzugten Ausführungsform der Erfindung bedeutet R¹ Methyl. X steht vorzugsweise für ein Chloratom.

In einer weiteren bevorzugten Ausführungsform steht R² für einen C₁₀-C₁₈-Alkylrest, insbesondere für einen C₁₂-C₁₆-Alkylrest, speziell für eine Mischung aus C₁₂-, C₁₄- und C₁₆-Alkylresten.
In einer weiteren bevorzugten Ausführungsform steht R³ für einen C₈-C₁₆-Alkylrest, oder für Methyl. R³ steht besonders bevorzugt für Methyl, wenn R² einen C₁₂-C₁₈-Alkylrest bedeutet, insbesondere wenn R² einen C₁₄-Alkylrest bedeutet.

In einer weiteren bevorzugten Ausführungsform steht R⁴ für einen Methylrest. R⁵ bedeutet vorzugsweise Wasserstoff. R⁶ hat vorzugsweise die Bedeutung Wasserstoff. m steht vorzugsweise für 3 oder 4.

Das erfindungsgemäße Verfahren wird vorzugsweise bei Temperaturen von 100 bis 140°C durchgeführt. Besonders bevorzugt sind Temperaturen von 110 bis 130°C:

Das Verfahren wird vorzugsweise unter Ausschluß von Wasser unter trockenem Schutzgas durchgeführt. Der Wassergehalt der Solventien sollte vorzugsweise unter 0,1 % liegen. Gut geeignete Solventien sind Dipropylenglykolmonomethylether, Tripropylenglykolmonomethylether, Dimethyltetraglykol, Dimethylpolyglykol mit einer mittleren Molmasse von etwa 200 - 350 g/mol. Besonders gut geeignet sind Butyldiglykol, Methylpolyglykol mit einer mittleren Molmasse von etwa 200-350 g/mol, und insbesondere Methyltriglykol und/oder Methyltetraglykol. Diese Solventien sind mit den Siloxanquats kompatibel, unterstützen die Endanwendung als antimikrobielle Formulierungen, besitzen günstige ökotoxikologische Eigenschaften und besitzen nicht den Nachteil der leichten Entflammbarkeit und leichten Brennbarkeit. Die Umsetzung von Halogenalkyltrialkoxysilan mit tertiärem Amin kann in Anwesenheit der oben genannten Solventien erfolgen, aber bevorzugt ist die Umsetzung in Abwesenheit dieser Solventien und deren Zugabe erst nach erfolgter Reaktion bei einer Temperatur, bei der die Reaktionsprodukte noch flüssig sind. Bei der Herstellung der Siloxanquat-Formulierungen in Anwesenheit von Solventien kann es vorkommen, daß geringe Mengen Alkohol aus der Alkoxygruppe des Halogenalkyltrialkoxysilans abgespalten werden. Diese geringen Mengen Alkohol können, falls erwünscht, beispielsweise durch Abdestillieren oder Abstrippen im Vakuum abgetrennt werden.

Eine im Rahmen der vorliegenden Erfindung besonders bevorzugte Siloxanquat-Formulierung wird hergestellt durch Umsetzung von 3-Chlorpropyltrimethoxysilan und Tetradecyldimethylamin im Molverhältnis 1:1 bis 1:1,1 bei 120°C (± 10°C) ohne Solvens und Zugabe von Methyltriglykol nach erfolgter Umsetzung.

Die erfindungsgemäße Formulierung enthält mindestens eine Siloxanverbindung ("Siloxanquat"), die durch Reaktion der Verbindungen der Formeln 1 und 2 herstellbar ist, sowie einen Stoff gemäß Formel 3. In einer bevorzugten Ausführungsform betragen die Anteile von Siloxanquat und Stoff der Formel 3 von 20:80 bis 80:20 Gew.-%, bezogen auf das Gewicht der Formulierung. Insbesondere bevorzugt sind Anteile von 40:60 bis 60:40, und insbesondere 45:55 bis 55:45 Gew.-%.

Bei der Durchführung des erfindungsgemäßen Verfahrens entstehen Verbindungen der Formel 4 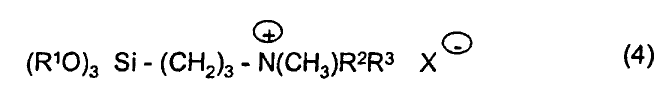 worin die Substituenten R¹, R², R³ und X die bereits oben angegebene Bedeutung haben.

Daneben entstehen nach dem erfindungsgemäßen Verfahren auch Verbindungen der Formel 5 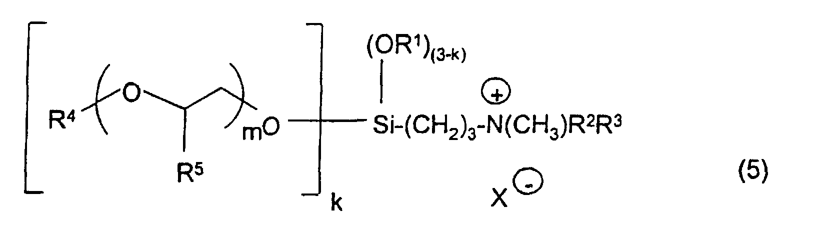 worin k 1, 2 oder 3 bedeutet, und die restlichen Substitutenten die bereits angegebene Bedeutung haben. Diese Verbindungen stellen einen weiteren Gegenstand der Erfindung dar.

Die erfindungsgemäßen Formulierungen sind geeignet, Oberflächen antimikrobiell auszurüsten. Bei diesen Oberflächen kann es sich beispielsweise um textile Gewebe, Glas, Holz, Cellulose, Metalle oder Kunststoffe handeln.
Verwendet man die erfindungsgemäße Formulierung für die Ausrüstung von Textilien, so wird sie vorzugsweise in einer Konzentration von 5 bis 100 g/l in Wasser gelöst, und auf die Textilien aufgezogen. Die Menge an Formulierung, welche auf den Textilien verbleibt, beträgt im allgemeinen 0,2 bis 3, vorzugsweise 0,5 bis 1 Gew.-%, bezogen auf das trockene Warengewicht.

Das Aufbringen der erfindungsgemäßen Formulierung kann mittels Foulardverfahren, Auszugsverfahren bei Badtemperaturen von 20 bis 70°C, Aufschäumung oder Aufsprühen erfolgen. Erfolgt das Aufbringen durch Aufsprühen, so beträgt die bevorzugte Konzentration der wäßrigen Lösung der erfindungsgemäßen Formulierung von 10 bis 300 g/l.

Die erfindungsgemäße Formulierung kann als wäßrige Lösung zur antimikrobiellen Ausrüstung von Fasern, Garnen oder Geweben aus Baumwolle oder Polyamid oder Polyester oder Wolle oder Mischungen aus diesen Fasertypen oder dieser Fasertypen zusammen mit Polyolefinfasern, wie vorzugsweise Polypropylen, durch das Foulardverfahren, durch das Auszugsverfahren, durch Sprühverfahren oder durch Verschäumen und Auftrag des Schaumes verwendet werden.

Die erfindungsgemäße Formulierung kann auch zusammen mit anderen Textilchemikalien, wie reine oder gemischte Carbonsäuren als Aviviermittel, wie Milch- und Weinsäure, wie Wachse, Stearate oder Silicon-Finisher als Nähgarngleitmittel, wie Silicone oder Paraffine als Hydrophobierungsmittel, Fluorcarbone mit oder ohne Zusatz von Melaminderivaten als Extender, Harze wie Melaminharze, Harnstoff/Formaldehydharze, Phenolharze oder Polyesterharze allein oder in Kombination eingesetzt werden.

Ebenfalls gute Ergebnisse werden mit den vorstehend beschriebenen Formulierungen dadurch erreicht, daß sie in Konzentrationen im Bereich von 5 g/l bis 100 g/l in Wasser gelöst werden und zusammen mit einem Schäumungsmittel wie einem Alkansulfonat, einem Alkylaminoxid, einem Alkylsulfat, einem Alkylethersulfat, und deren Salze, vorzugsweise einem Alkylsulfat verschäumt werden und daß der Schaum der, neben dem Schäumungsmittel die Formulierung alleine oder in Kombination mit anderen Textilchemikalien enthält, auf Textilien aufgetragen wird.

Auch lassen sich vorstehend beschriebenen Formulierungen mit guten Ergebnissen in Kombinationen mit anderen Textilchemikalien dadurch verwenden, daß sie als Lösungen, vorzugsweise wäßrige Lösungen, zusammen mit anderen Textilchemikalien wie Aviviermittel, Nähgamgleitmittel, Hydrophobierungsmittel, Fluorcarbonen mit oder ohne Extender, Harze für die Veränderung der Naßknittereigenschaften, der Schiebe- und Maschenfestausrüstung, permanente Griff-, Steif- und Fülleffekten und Flammschutzmitteln allein oder in Kombination eingesetzt werden.

Die Erfindung wird nun an Beispielen noch näher erläutert.

### Beispiel 1

500 g 3-Chlorpropyltrimethoxysilan und 620 g Tetradecyldimethylamin werden in einem 2 Liter-Rundkolben mit Innenthermometer, KPG-Rührer, Kühler und Druckhalteventil unter einer Stickstoffschutzgasatmosphäre und unter Ausschluß von Luftfeuchtigkeit vorgelegt. Die klare Reaktionsmischung wird bei 120°C 48 Stunden gerührt. Das gelbe Reaktionsprodukt wird abgekühlt und im noch heißen flüssigen Zustand (ca. 50°C) unter Rühren zu 1120 g Methyltriglykol mit einem Wassergehalt von unter 0,1 % gemischt. Die Ausbeute an Siloxanquat-Formulierung mit 50 % Solvens ist quantitativ.

Auch nach 24 Stunden Reaktionszeit erhält man bei noch unvollständigem Umsatz
eine sehr gut antimikrobiell wirksame Siloxanquat-Formulierung.

### Beispiel 2

119 g 3-Chlorpropyltrimethoxysilan und 160 g Tetradecyldimethylamin werden in einem 1 Liter-Rundkolben mit Innenthermometer, Magnetrührer, Kühler und Druckhalteventil unter einer Stickstoffschutzgasatmosphäre und unter Ausschluß von Luftfeuchtigkeit vorgelegt. Die klare Reaktionsmischung wird bei 140°C 24 Stunden gerührt. Nach Abkühlen auf 80 °C werden 420 g Methyltetraglykol mit einem Wassergehalt von unter 0,1 % zum orangegelben Reaktionsprodukt unter Rühren zudosiert. Die Ausbeute an Siloxanquat-Formulierung mit 60 % Solvens ist quantitativ.
Auch nach bereits 8 Stunden Reaktionszeit erhält man bei noch unvollständigem Umsatz eine sehr gut antimikrobiell wirksame Siloxanquat-Formulierung.

### Beispiel 3

119 g 3-Chlorpropyltrimethoxysilan und 135 g Genamin® LA 302 D, einer Mischung verschiedener Alkyldimethylamine mit der mittleren Molmasse von etwa 225 g/mol (Alkyl ist hauptsächlich Dodecyl und Tetradecyl) werden in einem 500 ml-Rundkolben mit Innenthermometer, Magnetrührer, Kühler und Druckhalteventil unter einer Stickstoffschutzgasatmosphäre und unter Ausschluß von Luftfeuchtigkeit vorgelegt. Die klare Reaktionsmischung wird bei 140°C 16 Stunden gerührt. Nach Abkühlen wird das noch warme, flüssige orangegelbe Reaktionsprodukt in 170 g Methyltriglykol mit einem Wassergehalt von unter 0,1 % eingerührt. Die Ausbeute an Siloxanquat-Formulierung mit 40 % Solvens ist quantitativ.

### Beispiel 4

50,0 g 3-Chlorpropyltrimethoxysilan, 56,8 g Genamin® LA 302 D (siehe Beispiel 3) und 107 g Methyltriglykol mit einem Wassergehalt von unter 0,1 % werden in einem 500 ml-Rundkolben mit Innenthermometer, Magnetrührer, Kühler und Druckhalteventil unter einer Stickstoffschutzgasatmosphäre und unter Ausschluß von Luftfeuchtigkeit bei 120°C insgesamt 23 Stunden gerührt. Nach Abkühlen auf 80°C werden geringe Mengen an Methanol, die sich während der Umsetzung gebildet haben, 3 Stunden im Vakuum bei 30 mbar und bei 80°C in einem Rotationsverdampfer abdestilliert. Die Ausbeute an Siloxanquat-Formulierung mit 50 % Solvens ist nahezu quantitativ.

### Beispiel 5

1000 g 3-Chlorpropyltrimethoxysilan, 1472 g Genamin® SH 302 D, einer Mischung verschiedener Alkyldimethylamine mit der mittleren Molmasse von etwa 292 g/mol (Alkyl ist hauptsächlich Octadecyl und Hexadecyl) werden in einem 4 Liter-Rundkolben mit Innenthermometer, KPG-Rührer, Kühler und Druckhalteventil unter einer Stickstoffschutzgasatmosphäre und unter Ausschluß von Luftfeuchtigkeit vorgelegt. Die klare Reaktionsmischung wird bei 100°C 168 Stunden gerührt. Das in dicken Schichten rötliche, in dünnen Schichten gelbe Reaktionsprodukt wird abgekühlt und im noch heißen flüssigen Zustand (ca. 60°C) unter Rühren zu 2470 g Dipropylenglykolmonomethylether gemischt. Die Ausbeute an Siloxanquat-Formulierung mit 50 % Solvens ist quantitativ.

### Beispiel 6

990,8 Gramm Leitungswasser werden vorgelegt, mit 0,2 Gramm Sandozin® NRW, einem Netzmittel versetzt. Zu dieser Lösung gibt man 9,0 Gramm eines erfindungsgemäßen Siloxanquats gemäß Beispiel 1 als 50% ige Lösung in Triethylenglykolmonomethylether zu. Die entstehende Lösung wird im Becken des Foulards vorgelegt und durch diese Lösung wird ein gewebtes Tuch aus Polyamid PA 6.6 mit einem Flächengewicht von 120 Gramm pro Quadratmeter gezogen und zwischen den Walzen so abgequetscht, daß das Tuch einen Flüssigkeitsanteil von 58,4% behält was einer Aufnahme von 0,52% der erfindungsgemäßen Formulierung, bezogen auf das trockene Warengewicht, ergibt. Das Tuch wird im Spannrahmen während zwei Minuten bei einer Temperatur von 110°C getrocknet. Ein Muster dieses Tuches wird wie nachfolgend beschrieben auf seine Wirkung gegen Bakterien geprüft. Zur Bestimmung der Waschbeständigkeit der antimikrobiellen Ausrüstung wird das Tuch bei 40°C im Verhältnis Waschlauge/Gewebe 30:1 gewaschen. Die Waschlauge enthält dabei 0,54 Gramm pro Liter polyethoxyliertes Nonylphenol mit neun bis zehn Ethylenoxideinheiten pro Nonylphenol, eingesetzt wurde Imbentin® N/52 der Kolb AG, Hedingen, Schweiz. Gewaschen wird während fünf Minuten. In der Wäscheschleuder wird die Waschlauge abgeschleudert, das Gewebe wird anschließend während rund zwei Minuten unter fließenden kalten Wasser gespült, nochmals abgeschleudert, das Spülen und das Abschleudern werden nochmals wiederholt und das Gewebe wird anschließend bei 80°C getrocknet.

Im Originalzustand, nach einer Wäsche, nach drei -, fünf -, zehn - und zwölf Wäschen werden Muster im Agar-Diffusionstest nach der Schweizer Norm SN 195 920 geprüft. Diese Prüfung wird gegen die Keime Staphylococcus aureus, Stamm ATCC 6538, gegen Pseudomonas aeruginosa, Stamm ATCC 15442, gegen Escherichia coli, Stamm ATCC 11229 und gegen Klebsiella pneumoniae, Stamm ATCC 4352 durchgeführt. Im Agar-Diffusionstest wird nach der Schweizer Norm SN 195 921 gegen Candida albicans, Stamm ATCC 10231 geprüft. Alle Muster zeigen sowohl im Originalzustand als auch nach den Wäschen, ausgewertet gemäß den zitierten Normen, eine gute Wirkung gegen alle aufgezählten Testkeime. Dies bedeutet keinen Bewuchs des Agars mit den Prüfkeimen unter den Testmustern bis hin zu der Ausbildung einer deutlichen Hemmzone um das Muster herum.

### Beispiel 7

15,0 Gramm Siliziumquat gemäß Beispiel 1 gelöst in 50% Triethylenglykolmonomethylether werden in 984,8 Gramm Wasser, versetzt mit 0,2 Gramm Sandozin NRW, gelöst und mittels Foulard auf ein Gewebe aus Baumwolle mit einem Flächengewicht von ungefähr 196 Gramm pro Quadratmeter so appliziert, dass man einen pick-up von 70% erhält. Das nasse Gewebe wird im Spannrahmen bei 110°C getrocknet und im Originalzustand und nach bis zu zwölf Wäschen nach der Methode von "Kokin-Boshu kako" gewaschen und anschließend im Agar-Diffusionstest nach der Norm SN 195 920 resp. 195 921 gegen die Keime Staphylococcus aureus, Stamm ATCC 6538, gegen Pseudomonas aeruginosa, Stamm ATCC 15442, gegen Escherichia coli, Stamm ATCC 11229, gegen Klebsiella pneumoniae, Stamm ATCC 4352 und gegen Candida albicans, Stamm ATCC 10231 geprüft. Praktisch alle Resultate zeigen eine gute Wirkung gegen die Testkeime bei der Auswertung nach den genannten Normen. Eine kleine Schwäche der Wirkung kann nur gegen den Keim Escherichia coli, Stamm ATCC 11229, nach über fünf Waschzyklen erkannt werden. Aber selbst hier ist eine deutliche Hemmung des Bakterienwachstums gegenüber einem nicht behandelten Stück Baumwollstoff erkennbar.
Diese Resultate sind überraschenderweise zum Teil deutlich besser als die Resultate, die mit Requat (Handelsmarke der Sanitized Inc.), einem handelsüblichen Produkt, das als siliziumfunktionale quarternäre Ammoniumverbindung 42% Didecylmethyl-3-(trimethoxysilyl)-propylammoniumchlorid gelöst in Methanol enthält.

### Beispiel 8

Auf Gewebe aus Polyester mit einem Flächengewicht von ungefähr 100 Gramm pro Quadratmeter wird mit der im Beispiel 6 beschriebenen Foulardmethode eine wäßrige Lösung mit 0,2 Gramm pro Liter Sandozin NRW und 7 Gramm pro Liter der 50% igen Siliziumquatlösung gemäß Beispiel 1 in Triethylenglykolmonomethylether so appliziert, daß ein pick-up von 75,2% erreicht wird. Diese Muster werden sowohl im Originalzustand als auch nach Wäschen, die so durchgeführt wurden, wie im Beispiel 6 beschrieben wurde, nach den Schweizerischen Normen SN 195 920 resp. 195 921 gegen die Keime, die im Beispiel 6 spezifiziert wurden, geprüft. Alle erhaltenen Testresultate zeigten, gegen alle im Beispiel 6 aufgezählten Testkeime, gute antimikrobielle Wirkung.

### Beispiel 9

Auf Gewebe aus Wolle mit einem Flächengewicht von ungefähr 160 Gramm pro Quadratmeter wird mit der im Beispiel 6 beschriebenen Foulardmethode eine wäßrige Lösung mit 0,2 Gramm pro Liter Sandozin NRW und 7 Gramm pro Liter der 50% igen Siliziumquatlösung gemäß Beispiel 1 in Triethylenglykolmonomethylether so appliziert, dass ein pick-up von 75,2% erreicht wird. Diese Muster werden sowohl im Originalzustand als auch nach Wäschen, die so durchgeführt wurden, wie im Beispiel 6 beschrieben wurde, nach den Schweizerischen Normen SN 195 920 resp. 195 921 gegen die Keime, die im Beispiel 6 spezifiziert wurden, geprüft. Alle erhaltenen Testresultate zeigten, gegen alle im Beispiel 6 aufgezählten Testkeime, gute antimikrobielle Wirkung.

### Beispiel 10

35,5 Gramm eines Gewebes aus Baumwolle werden 20 Minuten bei 40°C in 706 Milliliter einer wäßrigen Flotte, die 0,18 Gramm der erfindungsgemäßen Siloxanquatlösung gemäß Beispiel 1 in Triethylenglykolmonomethylether enthält, behandelt. Nachher wird die Flotte abgeschleudert und das Gewebe bei 80°C getrocknet. Diese Muster werden sowohl im Originalzustand als auch nach Wäschen, die so durchgeführt wurden, wie im Beispiel 6 beschrieben wurde, nach den Schweizerischen Normen SN 195 920 resp. 195 921 gegen die Keime, die im Beispiel 6 spezifiziert wurden, geprüft. Alle erhaltenen Testresultate zeigten, gegen alle im Beispiel 6 aufgezählten Testkeime, gute antimikrobielle Wirkung.

Wie in diesem Beispiel beschrieben werden Gewebe aus Polyamid, Polyester und Wolle jeweils mit 0,5% der erfindungsgemäßen Formulierung ausgerüstet und im Originalzustand und nach Wäschen im oben beschriebenen Test nach SN 195 920 und SN 195 921 gegen die genannten Keime geprüft. Alle Muster erreichen dabei gute antimikrobielle Ergebnisse.

### Beispiel 11

Ein Liter wäßrige Flotte enthaltend 7,5 Gramm der erfindungsgemäßen Formulierung gemäß Beispiel 1, 40,0 Gramm Nuva® CSF, einem Fluorcarbon des Typs Perfluoralkylacrylcopolymerisat von Clariant, 0,2 Gramm Sandozin NRW, einem Rapidnetzer von Clariant, wird angesetzt. Mittels dem Foulardverfahren wird diese Flotte auf ein Gewebe aus Baumwolle resp . auf ein Gewebe aus Polyamid so appliziert, daß von der erfindungsgemäßen Formulierung 0,52% und vom Fluorcarbon 2,77% bei dem Gewebe aus Baumwolle resp. 2,46 % bei dem Gewebe aus Polyamid aufgetragen werden. Die Gewebe werden anschließend je 2 Minuten auf dem Spannrahmen bei 110°C getrocknet und anschließend für 5 Minuten bei 140°C kondensiert. Diese Gewebe werden anschließend im Spray-Test nach AATCC 22 geprüft. Das so ausgerüstete Baumwollgewebe erreicht dabei einen Wert von 90 was gegenüber dem Gewebe, das nur mit dem Fluorcarbon behandelt worden ist einen wenig schwächeren Wert ergibt. Das Gewebe aus Polyamid erreicht im gleichen Test einen Wert von 100, was dem Wert des Gewebes entspricht, das nur mit dem Fluorcarbon ausgerüstet wird. Im Oleo-Test nach AATCC 118 erreichen beide Gewebe sowohl nur mit dem Fluorcarbon als auch mit dem Fluorcarbon zusammen mit der erfindungsgemäßen Formulierung die sehr gute Note 6. Im Agar-Diffusionstest nach SN 195 920 gegen die Bakterienstämme Staphylococcus aureus, Stamm ATCC 6538 und gegen Klebsiella pneumoniae, Stamm ATCC 4352 werden ausgezeichnete Werte mit deutlicher Hemmzone erreicht und gegen Escherichia coli, Stamm ATCC 11229 ergibt sich eine deutliche Wachstumshemmung, nämlich einen Bewuchs von unter 5% der Musterfläche.

### Beispiel 12

Ein Liter wäßrige Flotte enthaltend 7,5 Gramm der erfindungsgemäßen Formulierung gemäß Beispiel 1, 30,0 Gramm Sandolub® NV, einem Nähbarkeitsverbesserer von Clariant auf der Basis von aliphatischen Kohlenwasserstoffen, die in Wasser dispergiert sind, 0,5 Gramm Sandozin NRW, einem Rapidnetzer von Clariant, wird angesetzt. Mittels dem Foulardverfahren wird diese Flotte auf ein Gewebe aus Baumwolle resp . auf ein Gewebe aus Polyamid so appliziert, daß von der erfindungsgemäßen Formulierung 0,52 % und vom Nähbarkeitsverbesserer 2,0% auf den Geweben aufgetragen sind. Die Gewebe werden anschließend für zwei Minuten bei 110°C auf dem Spannrahmen getrocknet. Die Prüfung auf die antimikrobiellen Werte nach SN 195 920 gab gegen alle Bakterien, die im Beispiel 6 genannt wurden, ausgezeichnete Werte, die zwischen keinem Bewuchs und deutlichen Hemmzonen von einigen Millimetern schwankten.

### Beispiel 13

Ein Liter wäßrige Flotte enthaltend 7,5 Gramm der erfindungsgemäßen Formulierung gemäß Beispiel 1, 30,0 Gramm Sandoperm® MEW, einem modifizierten Polysiloxan, ein Weichmacher von Clariant, 0,5 Gramm Sandozin NRW, einem Rapidnetzer von Clariant, wird angesetzt. Mittels dem Foulardverfahren wird diese Flotte auf ein Gewebe aus Baumwolle resp . auf ein Gewebe aus Polyamid so appliziert, daß von der erfindungsgemäßen Formulierung 0,52 % und vom Weichmacher 2,0 % auf den Geweben aufgetragen sind. Die Gewebe werden anschließend für zwei Minuten bei 130°C auf dem Spannrahmen getrocknet. Die Prüfung auf die antimikrobiellen Werte nach SN 195 920 gab gegen alle Bakterien, die im Beispiel 6 genannt wurden, ausgezeichnete Werte, die zwischen keinem Bewuchs und deutlichen Hemmzonen von einigen Millimetern schwankten.

### Beispiel 14

Ein Liter wäßrige Flotte enthaltend 7,5 Gramm der erfindungsgemäßen Formulierung gemäß Beispiel 1, 200,0 Gramm Pekoflam® OP, einem Flammschutzmittel von Clariant auf der Basis eines organischen Salzes, 0,5 Gramm Sandozin NRW, einem Rapidnetzer von Clariant, wird angesetzt. Das Bad hat einen pH von 4,5 und wird so eingesetzt. Mittels dem Foulardverfahren wird diese Flotte auf ein Gewebe aus Baumwolle resp . auf ein Gewebe aus Polyamid so appliziert, daß von der erfindungsgemäßen Formulierung 0,5 % und vom Flammschutzmittel 13,5 % auf den Geweben aufgetragen sind. Die Gewebe werden anschließend für zwei Minuten bei 120°C auf dem Spannrahmen getrocknet. Die Prüfung auf die antimikrobiellen Werte nach SN 195 920 gab gegen alle Bakterien, die im Beispiel 6 genannt wurden, ausgezeichnete Werte, die zwischen keinem Bewuchs und deutlichen Hemmzonen von einigen Millimetern schwankten.

### Beispiel 15

Ein Liter wäßrige Flotte enthaltend 7,5 Gramm der erfindungsgemäßen Formulierung gemäß Beispiel 1,40,0 Gramm Appretan® EM, einem Polyvinylacetat das als Steifappretur eingesetzt wird, 0,5 Gramm Sandozin NRW, einem Rapidnetzer von Clariant, wird angesetzt. Mittels dem Foulardverfahren wird diese Flotte auf ein Gewebe aus Baumwolle resp . auf ein Gewebe aus Polyamid so appliziert, dass von der erfindungsgemäßen Formulierung 0,5 % und vom Appreturmittel rund 2,5 % auf den Geweben aufgetragen sind. Die Gewebe werden anschließend für zwei Minuten bei 120°C auf dem Spannrahmen getrocknet. Die Prüfung auf die antimikrobiellen Werte nach SN 195 920 gab gegen alle Bakterien, die im Beispiel 6 genannt wurden, ausgezeichnete Werte, die zwischen keinem Bewuchs und deutlichen Hemmzonen von einigen Millimetern schwankten.

### Beispiel 16

Zu 480 Milliliter Wasser gibt man 0,05 Gramm der erfindungsgemäßen Formulierung gemäß Beispiel 1 und 4,8 Gramm eines Filterpapiers. Das Papier läßt man bei 25 bis 30°C für 20 Minuten in der Flotte und trocknet es anschließend bei 80°C. Die Prüfung auf die antimikrobiellen Werte nach SN 195 920 gab gegen alle Bakterien, die im Beispiel 6 genannt wurden, ausgezeichnete Werte, die zwischen keinem Bewuchs und deutlichen Hemmzonen von einigen Millimetern schwankten.

### Tabellen mit den antimikrobiellen Resultaten aus den Beispielen:

Bei den nachfolgend aufgeführten Resultaten bedeuten Ziffern die Hemmzonen in Millimetern, die im Agar-Diffusionstest erreicht worden sind. Diese Hemmzonen sind nicht unbedingt identisch beim gleichen Test wieder erreichbar, da es sich um biologische Systeme mit sehr vielen Parametern handelt Die Aussage ist so zu interpretieren, daß die Ziffern 0 und größer bedeuten, daß das Muster selbst keinerlei Bewuchs mit den genannten Keimen aufweist und somit in der getesteten Form vollständig gegen Befall und Bewuchs durch den entsprechenden Keim geschützt ist. Der Buchstabe s bedeutet, dass das entsprechende Muster im durchgeführten Test einen schwachen Bewuchs bis zu 5 % der Musterfläche mit dem entsprechenden Keim aufwies. Dies bedeutet in der Praxis eine deutliche Wachstumshemmung und m bedeutet, dass das Muster einen mittleren Bewuchs von über 5 % bis 40 % der Fläche aufwies. Dieser Wert ist für einen antimikrobiellen Schutz ungenügend.

### Resultate zu Beispiel 6 (Foulardapplikation auf Polyamid):

| Keim | Original | 1 Wäsche | 3 Wäschen | 5 Wäschen | 10 Wäschen | 12 Wäschen |
|---|---|---|---|---|---|---|
| Staphylococcus aureus | 5 | 3 | 2 | 1 | 0 | 0 |
| Pseudomonas aeruginosa | 0 | 0 | 0 | 0 | 0 | 0 |
| Escherichia coli | 0 | 0 | 0 | 0 | 0 | 0 |
| Klebsiella pneumoniae | 2 | 0 | 0 | 0 | 0 | 0 |
| Candida albicans | 8 | 4 | 4 | 3 | 0 | 0 |

### Resultate zu Beispiel 7 (Foulardapplikation auf Baumwolle):

| Keim | Original | 1 Wäsche | 3 Wäschen | 5 Wäschen | 10 Wäschen | 12 Wäschen |
|---|---|---|---|---|---|---|
| Staphylococcus aureus | 6 | 5 | 3 | 2 | 2 | 1 |
| Pseudomonas aeruginosa | 0 | 0 | 0 | 0 | 0 | 0 |
| Escherichia coli | 0 | 0 | 0 | 0 | 5 | s |
| Klebsiella pneumoniae | 0 | 0 | 0 | 0 | 0 | 0 |
| Candida albicans | 0 | 0 | 0 | 0 | 0 | 0 |

### Gleiche Ausrüstung und Tests mit dem Requat:

| Keim | Original | 1 Wäsche | 3 Wäschen | 5 Wäschen | 10 Wäschen | 12 Wäschen |
|---|---|---|---|---|---|---|
| Staphylo-coccus aureus | 2 | 2 | 1 | 1 | 1 | 1 |
| Pseudomonas aeruginosa | 0 | 0 | 0 | 0 | s | s |
| Escherichia coli | 0 | 0 | s | s | s | m |
| Klebsiella pneumoniae | 1 | 1 | 0 | 0 | 0 | s |
| Candida albicans | 4 | 4 | 3 | 3 | 2 | 2 |

Bei diesem Beispiel ist der antimikrobielle Leistungsunterschied zwischen der erfindungsgemäßen Formulierung und einem marktgängigen Produkt sehr deutlich. Dieser Aspekt kommt noch zu den großen Vorteilen, die das Produkt bei der Applikation auf Grund seiner Zusammensetzung schon bietet.

### Resultate zu Beispiel 8 (Foulardapplikation auf Polyester):

| Keim | Original | 5 Wäschen | 10 Wäschen | 12 Wäschen |
|---|---|---|---|---|
| Staphylo-coccus aureus | 6 | 0 | 0 | 0 |
| Pseudomonas aeruginosa | 0 | 0 | 0 | 0 |
| Escherichia coli | 0 | 0 | 0 | 0 |
| Klebsiella pneumoniae | 5 | 0 | 0 | 0 |
| Candida albicans | 10 | 1 | 0 | 0 |

### Resultate zu Beispiel 9 (Foulardapplikation auf Wolle):

| Keim | Original | 5 Wäschen | 10 Wäschen | 12 Wäschen |
|---|---|---|---|---|
| Staphylo-coccus aureus | 2 | 0 | 0 | 0 |
| Pseudomonas aeruginosa | 0 | 0 | 0 | 0 |
| Escherichia coli | 0 | 0 | 0 | 0 |
| Klebsiella pneumoniae | 0 | 0 | 0 | 0 |
| Candida albicans | 5 | 0 | 0 | 0 |

### Resultate zu Beispiel 10 (Auszug auf Baumwolle):

| Keim | Original | 5 Wäschen | 10 Wäschen | 12 Wäschen |
|---|---|---|---|---|
| Staphylo-coccus aureus | 5 | 5 | 3 | 2 |
| Pseudomonas aeruginosa | 0 | 0 | 0 | 0 |
| Escherichia coli | 0 | 0 | 0 | 0 |
| Klebsiella pneumoniae | 2 | 0 | 0 | 0 |
| Candida albicans | 8 | 6 | 3 | 2 |

### Resultate zu Beispiel 10 (Auszug auf Polyamid):

| Keim | Original | 5 Wäschen | 10 Wäschen | 12 Wäschen |
|---|---|---|---|---|
| Staphylo-coccus aureus | 2 | 0 | 0 | 0 |
| Pseudomonas aeruginosa | 0 | 0 | 0 | 0 |
| Escherichia coli | 0 | 0 | 0 | 0 |
| Klebsiella pneumoniae | 0 | 0 | 0 | 0 |
| Candida albicans | 3 | 0 | 0 | 0 |

### Resultate zu Beispiel 10 (Auszug auf Polyester):

| Keim | Original | 5 Wäschen | 10 Wäschen | 12 Wäschen |
|---|---|---|---|---|
| Staphylo-coccus aureus | 0 | 0 | 0 | 0 |
| Pseudomonas aeruginosa | 0 | 0 | 0 | 0 |
| Escherichia coli | 0 | 0 | 0 | 0 |
| Klebsiella pneumoniae | 0 | 0 | 0 | 0 |
| Candida albicans | 1 | 0 | 0 | 0 |

### Resultate zu Beispiel 10 (Auszug auf Wolle):

| Keim | Original | 5 Wäschen | 10 Wäschen | 12 Wäschen |
|---|---|---|---|---|
| Staphylo-coccus aureus | 0 | 0 | 0 | 0 |
| Pseudomonas aeruginosa | 0 | 0 | 0 | 0 |
| Escherichia coli | 0 | 0 | 0 | 0 |
| Klebsiella pneumoniae | 0 | 0 | 0 | 0 |
| Candida albicans | 2 | 0 | 0 | 0 |

### Resultate zu Beispiel 11 (Applikation zusammen mit Nuva CSF):

| | Baumwolle | Polyamid |
|---|---|---|
| Staphylococcus aureus | 4 | 0 |
| Escherichia coli | s | s |
| Pseudomonas aeruginosa | m | m |
| Klebsiella pneumoniae | 1 | 0 |
| Candida albicans | 0 | m |

### Resultate zu den weiteren Beispielen 12 bis 16:

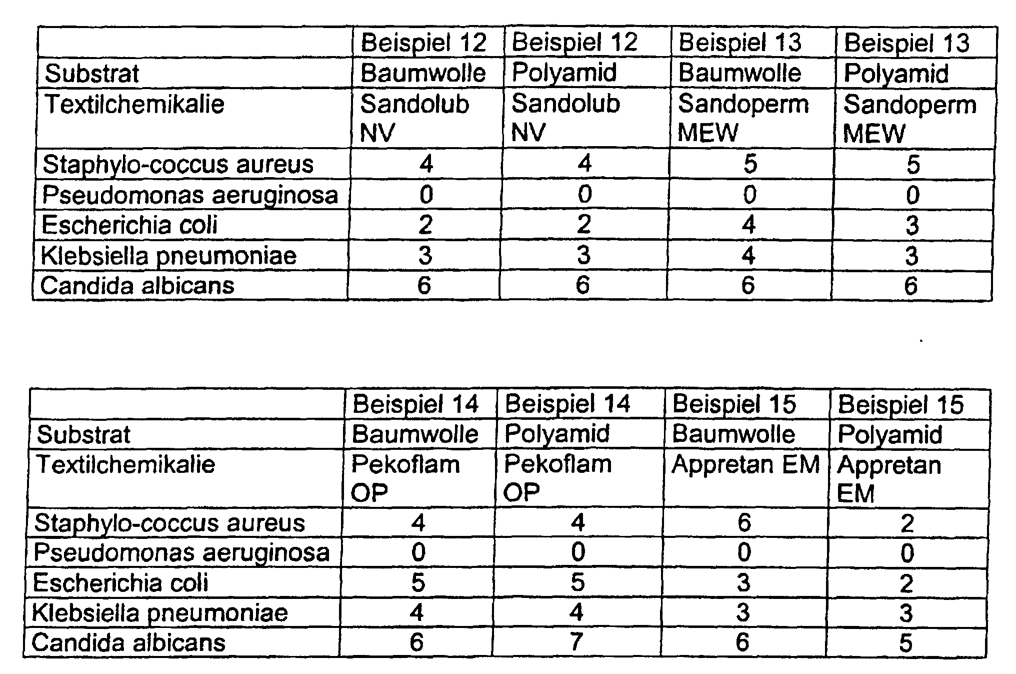

mit Filterpapier, Beispiel 16

| Staphylococcus aureus | Pseudomonas aeruginosa | Escherichia coli | Klebsiella pneumoniae |
|---|---|---|---|
| 2 | 0 | 0 | 0 |

## Patentansprüche

1. Verfahren zur Herstellung einer antimikrobiellen Formulierung, umfassend die Reaktion einer Verbindung der Format 1
(R¹O)₃Si - (CH₂)₃ - X (1)
mit einer Verbindung der Formel 2
(H₃C)NR²R³ (2)
worin
R¹ C₁-C₄-AIkyl
R² C₈-C₂₀-Alkyl
R³ Methyl oder C₈-C₂₀-AlkyI
X Cl, Br bedeuten,
unter Ausschluß von lodiden im Molverhältnis von (1): (2) = 1:0,9 bis 1:1,4, **gekennzeichnet durch** die Durchführung der Reaktion in einem Lösungsmittel, welches der Formel 3 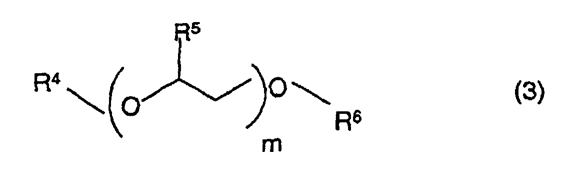 entspricht, worin
R⁴ C₁-C₄-Alkyl
R⁵ H oder Methyl
R⁶ H oder Methyl
m 2, 3, 4 oder 5,
wobei für m = 2 aber R⁴ und R⁶ nicht gleichzeitig Methyl bedeuten, und dieses Lösungsmittel nach der Reaktion nicht abgetrennt wird, oder die Zugabe dieses Lösungsmittels nach der Reaktion.

2. Verfahren gemäß Anspruch 1, worin R¹ für Methyl steht.

3. Verfahren gemäß Anspruch 1 und/oder 2, worin R² für C₁₀-C₁₈-Alkyl steht.

4. Verfahren gemäß einem oder mehreren der Ansprüche 1 bis 3, worin R³ für C₈-C₁₆-Atkyt steht.

5. Verfahren gemäß Anspruch 1 und/oder 2, worin R² für C₁₄-Alkyt und R³ für Methyl steht.

6. Verfahren gemäß einem oder mehreren der Ansprüche 1 bis 5, worin R⁴ für Methyl steht.

7. Verfahren gemäß einem oder mehreren der Ansprüche 1 bis 6, worin R⁵ für Wasserstoff steht.

8. Verfahren gemäß einem oder mehreren der Ansprüche 1 bis 7, worin R⁶ für H steht.

9. Verfahren gemäß einem oder mehreren der Ansprüche 1 bis 8, worin m für 3 oder 4 steht.

10. Antimikrobielle Formulierung, hergestellt gemäß dem Verfahren nach einem oder mehreren der Ansprüche 1 bis 9.

11. Verwendung von Formulierungen gemäß Anspruch 10 zur antimikrobiellen Ausrüstung von Oberflächen.

## Claims

1. A process for preparing an antimicrobial formulation by reacting a compound of the formula 1
(R¹O)₃Si - (CH₂)₃- X (1)
with a compound of the formula 2
(H₃C)NR²R³ (2)
where
R¹ is C₁-C₄-alkyl
R² is C₈-C₂₀-alkyl
R³ is methyl or C₈-C₂₀-alkyl
X is Cl or Br,
excluding iodides, in a molar ratio of (1):(2) = 1:0.9 to 1:1.4,
which comprises conducting said reaction in a solvent conforming to the formula 3 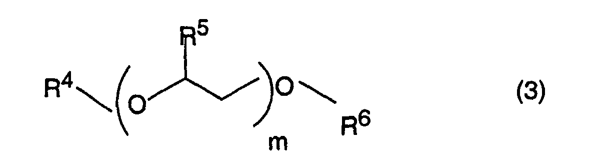 where
R⁴ is C₁-C₄-alkyl
R⁵ is H or methyl
R⁶ is H or methyl
m is 2, 3, 4 or 5,
subject to the proviso that when m is 2 R⁴ and R⁶ are not both methyl, and not removing this solvent after said reaction, or adding this solvent after said reaction.

2. The process of claim 1, wherein R¹ is methyl.

3. The process of claim 1 and/or 2, wherein R² is C₁₀-C₁₈-alkyl.

4. The process of one or more of claims 1 to 3, wherein R³ is C₈-C₁₆-alkyl.

5. The process of claim 1 and/or 2, wherein R² is C₁₄-alkyl and R³ is methyl.

6. The process of one or more of claims 1 to 5, wherein R⁴ is methyl.

7. The process of one or more of claims 1 to 6, wherein R⁵ is hydrogen.

8. The process of one or more of claims 1 to 7, wherein R⁶ is H.

9. The process of one or more of claims 1 to 8, wherein m is 3 or 4.

10. An antimicrobial formulation obtained by the process of one or more of claims 1 to 9.

11. The use of formulations as claimed in claim 10 for antimicrobial finishing of surfaces.

## Revendications

1. Procédé de préparation d'une formulation antimicrobienne, qui comprend la réaction d'un composé de formule 1
(R¹O)₃Si- (CH₂)₃-X (1)
avec un composé de formule 2
(H₃C)NR²R³ (2)
où
R¹ est un groupe alkyle en C₁-C₄,
R² est un groupe alkyle en C₈-C₂₀,
R³ est le groupe méthyle ou un groupe alkyle en C₈C₂₀,
X est Cl, Br,
à l'exception des iodures, selon un rapport en moles (1):(2) = 1:0,9 à 1:1,4,
**caractérisé par** la mise en oeuvre de la réaction dans un solvant correspondant à la formule 3 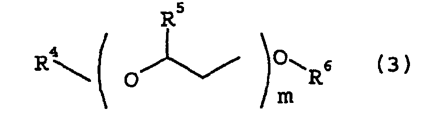 dans laquelle
R⁴ est un groupe alkyle en C₁-C₄,
R⁵ est un atome d'hydrogène ou le groupe méthyle,
R⁶ est un atome d'hydrogène ou le groupe méthyle,
m vaut 2, 3, 4 ou 5,
R⁴ et R⁵ ne représentant cependant pas simultanément des radicaux méthyle quand m = 2, ce solvant n'étant pas isolé après la réaction, ou encore l'addition de ce solvant après la réaction.

2. Procédé selon la revendication 1, dans lequel R¹ est le groupe méthyle.

3. Procédé selon la revendication 1 et/ou 2, dans lequel R² est un groupe alkyle en C₁₀-C₁₈.

4. Procédé selon l'une ou plusieurs des revendications 1 à 3, dans lequel R³ est un groupe alkyle en C₈-C₁₆.

5. Procédé selon la revendication 1 et/ou 2, dans lequel R² est un groupe alkyle en C₁₄ et R³ est le groupe méthyle.

6. Procédé selon l'une ou plusieurs des revendications 1 à 5, dans lequel R⁴ est le groupe méthyle.

7. Procédé selon l'une ou plusieurs des revendications 1 à 6, dans lequel R⁵ est un atome d'hydrogène.

8. Procédé selon l'une ou plusieurs des revendications 1 à 7, dans lequel R⁶ est H.

9. Procédé selon l'une ou plusieurs des revendications 1 à 8, dans lequel m vaut 3 ou 4.

10. Formulation antimicrobienne préparée par le procédé selon l'une ou plusieurs des revendications 1 à 9.

11. Utilisation de formulations selon la revendication 10 pour le traitement antimicrobien de surfaces.
